# EUROPEAN PATENT APPLICATION

(11) **EP 2 062 611 A1**
(43) Date of publication of application: **27.05.2009**
(21) Application number: 07792660.8
(22) Date of filing: 17.08.2007
(51) Int. Cl.: A61M 37/00

(54) **MICRONEEDLE AND MICRONEEDLE PATCH**

(30) Priority: 18.08.2006 JP 2006223601
(71) Applicant: Toppan Printing Co., Ltd., Taito-ku, Tokyo 110-0016 (JP)
(72) Inventor: TOMONO, Takao, 5-1, Taito 1-chome, Taito-ku Tokyo 110-0016 (JP)
(74) Representative: TBK-Patent
(86) International application number: PCT/JP2007/066044
(87) International publication number: WO 2008/020632

(57) **Abstract**

Insertion of a micro-needle into a living body is made easy. The micro-needle (121) includes first and second end sections (121a, 121b) arranged in a longitudinal direction and is made of a biocompatible and biodegradable material including chitin and/or chitosan.

## Description

### Technical Field

The present invention relates to a micro-needle patch applied, for example, to a surface of a living body.

### Background Art

Generally, transdermal administration of a drug to a living body includes application of a liquid or viscous body containing the drug to the skin. However, the applied drug is prone to be removed from the surface of the skin due to perspiration or contact. In addition, when the applied drug is intended to penetrate into the inner layer of the skin, the degree of penetration is difficult to control.

In this connection, use of a micro-needle array for administration of a drug is proposed. A micro-needle array has a structure in which micro-needles are arranged on a substrate. For example, JP-A 2003-238347 (KOKAI) describes a micro-needle array including a polymethylmethacrylate substrate and micro-needles of maltose formed thereon.

For administration of a drug with a micro-needle array, used is a micro-needle array whose micro-needles contain the drug, for example. To be more specific, such a micro-needle array is pressed against the skin to insert the micro-needles into the living body. In the case where the micro-needles contain a drug, by leaving the micro-needles in the living body, it is possible to prevent the drug from being removed from the living body due to perspiration, contact, etc. In addition, the degree of penetration of the drug can be controlled, for example, according to the lengths and/or density of the micro-needles.

A micro-needle array is required that the micro-needles are inserted into the living body with reliability. However, the present inventor has found out the following fact in the course of animal tests in achieving the present invention. That is, the micro-needles that contain maltose as a main component are difficult to insert into the living body.

### Disclosure of Invention

An object of the present invention is to provide a micro-needle that is easy to be inserted into the living body.

According to a first aspect of the present invention, there is provided a micro-needle comprising first and second end sections arranged in a longitudinal direction, and made of a biocompatible and biodegradable material including chitin and/or chitosan.

According to a second aspect of the present invention, there is provided a micro-needle patch, comprising a support layer with first and second main surfaces, and micro-needles each extending from the first main surface, each of the micro-needles being the micro-needle according to one of claims 1 to 10 supported by the first main surface at an end of the second end section.

### Brief Description of Drawings

FIG. 1 is a perspective view schematically showing a micro-needle patch according to an embodiment of the present invention;
FIG. 2 is a perspective view schematically showing the micro-needle patch shown in FIG. 1 provided with a protection member;
FIG. 3 is a perspective view schematically showing a part of the micro-needle patch shown in FIG. 1;
FIG. 4 is a perspective view schematically showing a micro-needle included in the structure shown in FIG. 3;
FIG. 5 is a perspective view schematically showing an example of modified micro-needle;
FIG. 6 is a perspective view schematically showing an example of modified micro-needle;
FIG. 7 is a perspective view schematically showing an example of modified micro-needle;
FIG. 8 is a perspective view schematically showing an example of modified micro-needle;
FIG. 9 is a perspective view schematically showing an example of modified micro-needle;
FIG. 10 is a perspective view schematically showing an example of modified micro-needle;
FIG. 11 is a perspective view schematically showing an example of modified micro-needle;
FIG. 12 is a perspective view schematically showing an example of modified micro-needle;
FIG. 13 is a perspective view schematically showing an example of modified micro-needle;
FIG. 14 is a flow-chart showing an example of a method for manufacturing a micro-needle patch;
FIG. 15 is a sectional view schematically showing a structure of a micro-needle employed in Example 1;
FIG. 16 is a sectional view schematically showing a structure of a micro-needle employed in Example 1;
FIG. 17 is a sectional view schematically showing a structure of a micro-needle employed in Example 1;
FIG. 18 is a sectional view schematically showing a structure of a micro-needle employed in Example 1;
FIG. 19 is a sectional view schematically showing a structure of a micro-needle employed in Example 1;
FIG. 20 is a sectional view schematically showing a structure of a micro-needle employed in Example 1;
FIG. 21 is a sectional view schematically showing a structure of a micro-needle employed in Example 2;
FIG. 22 is a sectional view schematically showing a structure of a micro-needle employed in Example 2; and
FIG. 23 is a graph showing the relationship between the insulin content and the strength of a micro-needle.

### Best Mode for Carrying Out the Invention

An embodiment of the present invention will be described below. In the drawings, the same reference symbols denote components having the same or similar functions and duplicate descriptions will be omitted.

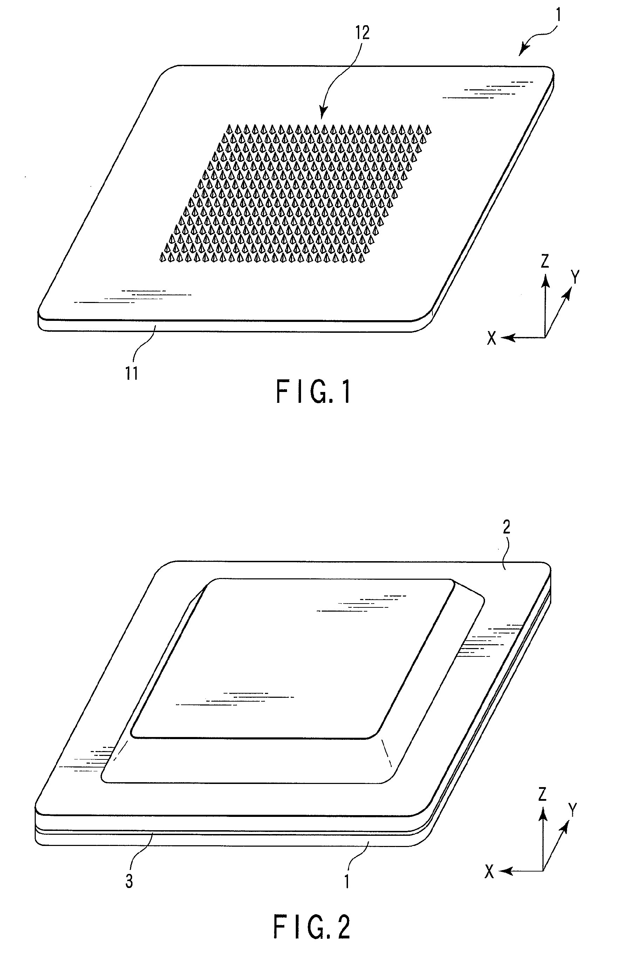

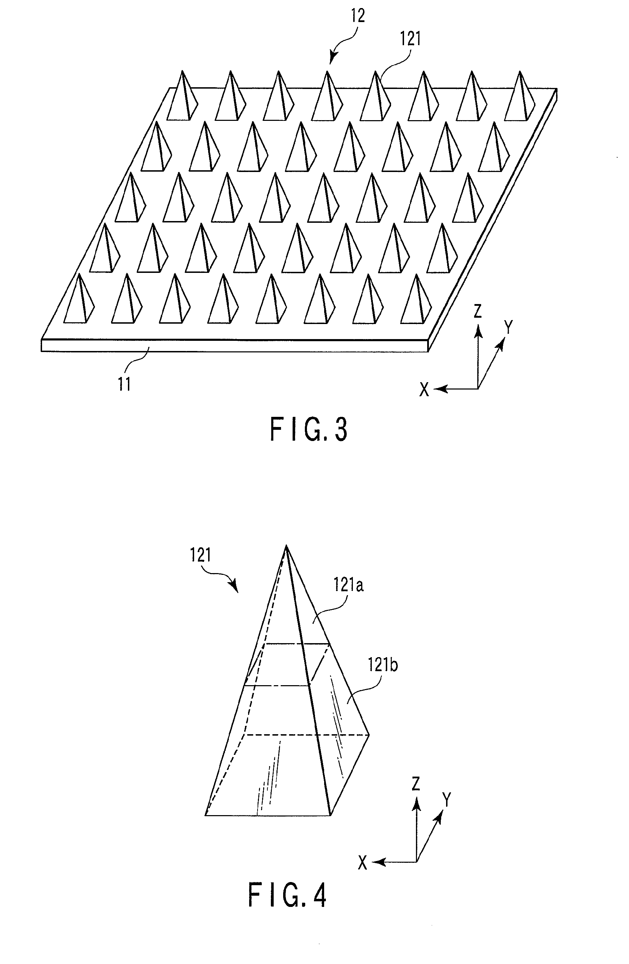

FIG. 1 is a perspective view schematically showing a micro-needle patch according to an embodiment of the present invention. FIG. 2 is a perspective view schematically showing the micro-needle patch shown in FIG. 1 provided with a protection member. FIG. 3 is a perspective view schematically showing a part of the micro-needle patch shown in FIG. 1. FIG. 4 is a perspective view schematically showing a micro-needle included in the structure shown in FIG. 3.

Note that in FIGS. 1 to 4, the X and Y directions are the directions parallel with a main surface of the micro-needle patch and perpendicular to each other. Note also that the Z direction is the direction perpendicular to the X and Y directions.

The micro-needle patch 1 shown in FIG. 1 includes a support layer 11 and a micro-needle array 12. The support layer 11 includes first and second main surfaces. The first main surface supports the micro-needle array 12.

Before using the micro-needle patch 1, the micro-needle array 12 is protected, for example, using the protection member 2 shown in FIG. 2. The protection member 2 shown in FIG. 2 is a plate-like molded article recessed at the position corresponding to the micro-needle array 12, and adhered to the support layer 11 via the adhesive layer 3. When the micro-needle patch 1 is used, it is removed from the protection member 2. Then, the micro-needle patch 1 is pressed against a living body such that the micro-needle array 12 is inserted therein.

Next, the constituents of the micro-needle patch 1 will be described in more detail.

The support member 11 shown in FIGS. 1 and 3 has a monolayer structure or multilayered structure. The support layer 11 may be rigid or flexible. As the material of the support layer 11, for example, organic polymer such as plastic, metal, glass or a mixture thereof may be used. When a multilayered structure is employed in the support layer 11, a part thereof may be a cloth or paper.

Typically, the main surface of the support layer 11 on the side of the micro-needle array 12 is made of a material including chitin and/or chitosan. Note that chitosan is a deacetylated product of chitin. Note also that "chitin and/or chitosan" refers to at least one of chitin and chitosan, and typically is chitosan or a mixture of chitin and chitosan. Hereinafter, "chitin and/or chitosan" is abbreviated to "chitin/chitosan".

As shown in FIG. 3, the micro-needle array 12 is composed of micro-needles 121. The micro-needles 121 extend from the first main surface of the support layer 11.

As shown in FIG. 4, each micro-needle 121 includes a first end section 121a and a second end section 121b arranged in a longitudinal direction. Note that in FIG. 4, the plane drawn in the alternate long and short dash line shows the boundary surface between the first end section 121a and the second end section 121b.

The first end section 121a has roughly a quadrangular pyramid shape. The second end section 121b has roughly a truncated quadrangular pyramid shape. The first end section 121a and the second end section 121b are equal in angles of inclinations of lateral faces. In addition, the lateral faces of the first end section 121a are flush with the lateral faces of the second end section 121b. That is, each micro-needle 121 has roughly a quadrangular pyramid shape whose base is parallel with the X and Y directions.

The micro-needles 121 are made of a biocompatible and biodegradable material including chitin/chitosan.

As shown in TABLE 1 below, chitin/chitosan has sufficiently high Young's modulus and tensile strength. Note that in TABLE 1 below, "PLA" denotes polylactic acid, and "PLGA" denotes a copolymer of polylactic acid and glucose. Note also that the numerical values in TABLE 1 below are only examples, and may slightly vary according molecular weight, etc.

**TABLE 1**

| Main component of material | Young's modulus (GPa) | Tensile strength (MPa) | Decomposition rate (half-life) |
|---|---|---|---|
| Chitin/chitosan | 6 | 60 | 2 weeks |
| PLA | 1.5-2.5 | 20-60 | 1 month-1 year |
| PLGA | 2-9 | 40-850 | 10 weeks-7 months |
| Mg | 45 | 230 | 2-3 weeks |
| Ti | 110 | 320 | - |
| SUS304 (injection needle) | 197 | 520 | - |

Skin of a living body has elasticity. For example, epidermis, dermis and subcutaneous tissue of a human have Young's moduli of about 0.14 MPa, about 0.080 MPa and about 0.034 MPa, respectively.

In order to insert a needle into the epidermis, the force stronger than the Young's modulus of the epidermis is necessary. In order to insert the needle into the epidermis with reliability, the force should be over about 100 times, preferably over about 1,000 times the Young's modulus of the epidermis. On the other hand, in order to withdraw the needle, the tensile strength of the needle should be, for example, 5 MPa or more, desirably 50 MPa or more.

As shown in TABLE 1 above, chitin/chitosan has a sufficient Young's modulus. Thus, the micro-needles 121 including chitin/chitosan can be easily inserted into a living body. Therefore, for example, when a predetermined amount of a feed substance is supported by surfaces of the micro-needles 121, the feed substance can be fed into the living body at almost the same amount as the design value.

In addition, as shown in TABLE 1 above, chitin/chitosan has a sufficient tensile strength. Therefore, the micro-needles 121 including chitin/chitosan resist breaking when they are withdrawn from the living body.

Furthermore, the micro-needles 121 are made of a biocompatible and biodegradable material. As shown in TABLE 1 above, chitin/chitosan degrades in short time in a living body. Thus, when a broken micro-needle 121 is left in a living body, the micro-needle 121 does not prevent the healing of a wound caused by pressing the micro-needle patch 1 against a surface of the living body.

In addition, chitin/chitosan has hemostatic and bactericidal properties. Therefore, the micro-needles 121 accelerate the stopping up of the wound caused by pressing the micro-needle patch 1 against a surface of the living body so as to prevent the invasion of viruses into the living body, and inhibit the growth of viruses in the living body. That is, the micro-needle 121 left in the living body encourages the healing of the wound caused by pressing the micro-needle patch 1 against a surface of the living body.

As the feed substance described above, for example, a bioactive substance that acts on a structural element of a living body, a bioinert substance that does not act on a structural element of a living body, or a mixture thereof can be used. As the bioactive substance, one or more substances that can cause a physiological change in a living body when administered to the living body, for example, drugs. As this drug, for example, insulin, ketamine, nitroglycerin, isosorbide dinitrate, estradiol, tulobuterol, nicotine, scopolamine or clonidine hydrochloride can be used. As the bioinert substance, for example, one or more substances used in cosmetics such as dye and humectant can be used.

The biocompatible and biodegradable material can further include another substance in addition to chitin/chitosan. As the substance that the biocompatible and biodegradable material can further include, for example, the above-described bioactive substance, bioinert substance or mixture containing one or more of them can be used.

The sum of chitin content and chitosan content in the biocompatible and biodegradable material is set, for example, at 50% by mass or more. When the sum of chitin content and chitosan content is small, Young's modulus and/or tensile strength of the micro-needles 121 may be insufficient.

In the projections of the micro-needle 121 onto planes perpendicular to the XY plane, the minimum angle of the tip of the first end section 121a is, for example, within a range of about 9° to about 53°, and typically within a range of about 15° to about 20°. In the case where the angle is small, the micro-needles 121 prone to be broken when the micro-needle array 12 or the micro-needle patch 1 is transported or when the micro-needle patch 1 is applied to a living body. In the case where the angle is large, a stronger force is necessary for inserting the micro-needles 121 into the surface of a living body as compared with the case where the angle is small. That is, in the case where the angle is large, it is difficult to smoothly insert the micro-needles 121 into the surface of a living body.

The dimension of the micro-needles 121 in the Z direction is, for example, within a range of about 20 µm to about 1.4 mm. As will be described below, the dimension can be determined according to the application of the micro-needle patch 1.

The skin of human has a three-layered structure of epidermis, dermis and subcutaneous tissue. The thickness of the epidermis is within a range of about 0.07 mm to about 0.2 mm. The thickness of the stratum corneum is about 0.02 mm. The thickness of the skin constituted by the epidermis and the dermis is within a range of about 1.5 mm to about 4 mm.

The feed substance such as the bioactive substance cannot penetrate into the body unless the substance reaches to the dermis. Thus, for such an application, the dimension of the micro-needles 121 in the Z direction is set, for example, at about 0.02 mm or more, and typically at about 0.2 mm or more. In order to insert the micro-needles 121 through the epidermis with reliability, the dimension of the micro-needles 121 in the Z direction is set, for example, at about 0.3 mm or more. In order to insert the micro-needles 121 through the skin with reliability, the dimension of the micro-needles 121 in the Z direction is set, for example, at about 4 mm or more.

The maximum dimension of the micro-needles 121 parallel with the XY plane is, for example, about 300 µm or less. The dimension can be determined, for example, in consideration of pain that the micro-needles 121 make the living body feel.

An injection needle having a thickness of 0.2 mm is commercially available as a painless needle. In order to make a human feel no pain, the maximum dimension of the micro-needles 121 parallel with the XY direction should be, for example, about 0.15 mm or less, and typically within a range of about 0.05 mm to about 0.07 mm.

Various modifications to the micro-needles 121 can be possible.

In the micro-needle 121 shown in FIG. 4, the first end section 121a has roughly a quadrangular pyramid shape. The first end section 121a may have another shape. For example, the first end section 121a may be a cylinder such as circular cylinder, elliptic cylinder and prism. The cylinder may be a right cylindrical body, an oblique cylindrical body or a truncated cylindrical body. However, the first end section 121a typically employs the structure in which it is tapered down from an end on the side of the second end section 121b to another end. In this case, the first end section 121a may be, for example, a cone such as circular cone, elliptic cone and pyramid. The cone may be a right cone, an oblique cone, a right truncated cone or an oblique truncated cone.

In the micro-needle 121 shown in FIG. 4, the second end section 121b has roughly a truncated quadrangular pyramid shape. The second end section 121b may have another shape. For example, the second end section 121b may be a cylinder such as circular cylinder, elliptic cylinder and prism. Alternatively, the second end section 121b may be tapered down from an end on the side of the first end section 121a to another end. In this case, the second end section 121b may be, for example, a truncated cone such as circular truncated cone, elliptic truncated cone and truncated pyramid. The truncated cone may be a right truncated cone or an oblique truncated cone. However, the second end section 121b typically employs the structure in which it is tapered down from an end on the side of the support layer 11 to another end. In this case, the second end section 121b may be, for example, a truncated cone such as truncated circular cone, truncated elliptic cone and truncated pyramid. The truncated cone may be a right truncated cone or an oblique truncated cone.

In the micro-needle 121 shown in FIG. 4, the micro-needle 121 has roughly a quadrangular pyramid shape whose base is parallel with the X and Y directions. The micro-needle 121 may have another shape. For example, the micro-needle 121 may have any shape obtained by combining the shape described for the first end section 121a with the shape described for the second end section 121b. However, the micro-needle 121 typically employs the structure in which it is tapered down from an end of the support layer 11 to another end. In this case, the micro-needle 121 may be, for example, a cone such as circular cone, elliptic cone and pyramid. The cone may be a right cone, an oblique cone, a right truncated cone or an oblique truncated cone. Alternatively, the micro-needle 121 may have the shape obtained by combining the first end section 121a having a cone shape with the second end section 121b having a cylindrical shape.

At least one of the micro-needles 121 may have a symmetry axis parallel with the longitudinal direction thereof. Such a micro-needle 121 resists breaking when it is pressed against the surface of a living body.

At least one of the micro-needles 121 may be asymmetric. For example, at least one of the micro-needles 121 may have no symmetrical axis parallel with the longitudinal direction thereof. In this case, the micro-needle 121 is prone to be broken when applied with a force in a direction crossing the Z direction as compared with the case where the micro-needle 121 has a symmetrical axis parallel with the Z direction.

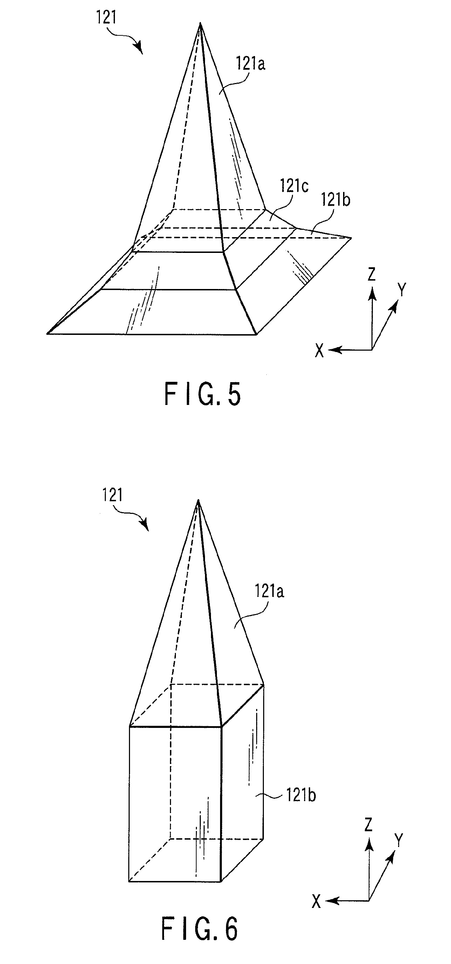

FIGS. 5 to 13 are perspective views schematically showing examples of modified micro-needle.

The micro-needle 121 shown in FIG. 4 has the structure in which it is tapered down from an end on the side of the support layer 11 to another end. The first end section 121a has a quadrangular pyramid shape. The second end section 121b has a truncated quadrangular pyramid shape. The angles that the lateral faces of the first end section 121a make with the Z direction are smaller than the angles that the lateral faces of the second end section 121b make with the Z direction.

As such, the first end section 121a and the second end section 121b may be different from each other in the angles of inclinations of lateral faces. When such a structure is employed in which the angles that the lateral faces of the first end section 121a make with the Z direction are smaller than the angles that the lateral faces of the second end section 121b make with the Z direction, a micro-needle that is easy to insert into the surface of a living body and resists breaking at the position of the second end section 121b can be obtained. When such a structure is employed in which the angles that the lateral faces of the first end section 121a make with the Z direction are larger than the angles that the lateral faces of the second end section 121b make with the Z direction, a micro-needle that resists breaking over the entire length thereof can be obtained.

The micro-needle 121 shown in FIG. 5 further includes a middle section 121c interposed between the first end section 121a and the second end section 121b. The middle section 121c has a truncated quadrangular pyramid shape. The angles that the lateral faces of the middle section 121c make with the Z direction are larger than the angles that the lateral faces of the first end section 121a make with the Z direction and smaller than the angles that the lateral faces of the second end section 121b make with the Z direction.

As such, the micro-needle 121 may further includes the middle section 121c having a truncated cone or columnar shape different in the angles of inclinations of lateral faces from the first end section 121a and the second end section 121b. In the case where the angles of inclinations of lateral faces of the middle section 121c are between the angles of inclinations of lateral faces of the first end section 121a and the angles of inclinations of lateral faces of the second end section 121b, the physical properties of the micro-needle 121 can be gradually changed in the Z direction. When the structure shown in FIG. 5 is employed, the strength at and near the second end section 121b can be increased. Therefore, breaking of the micro-needle 121 at and near the second end section 121b can be suppressed.

The inclinations of the middle section 121c with respect to the Z direction may be smaller than the inclinations of the first end section 121a with respect to the Z direction and the inclinations of the second end section 121b with respect to the Z direction. For example, it is possible that the first end section 121a is a cone or truncated cone, the second end section 121b is a truncated cone, and the middle section 121c is a columnar. Such a structure is advantageous in suppressing breaking of the micro-needle 121 at and near the second end section 121b, and is useful when the tip of the micro-needle 121 must reach to a position far from the surface of a living body.

The micro-needle 121 shown in FIG. 6 has the structure in which it is tapered down from an end on the side of the support layer 11 to another end. The first end section 121a has a quadrangular pyramid shape. The second end section 121b has a quadrangular prism shape. As such, the micro-needle 121 whose second end section 121b has a columnar shape is useful when the tip of the micro-needle 121 must reach to a position far from the surface of a living body.

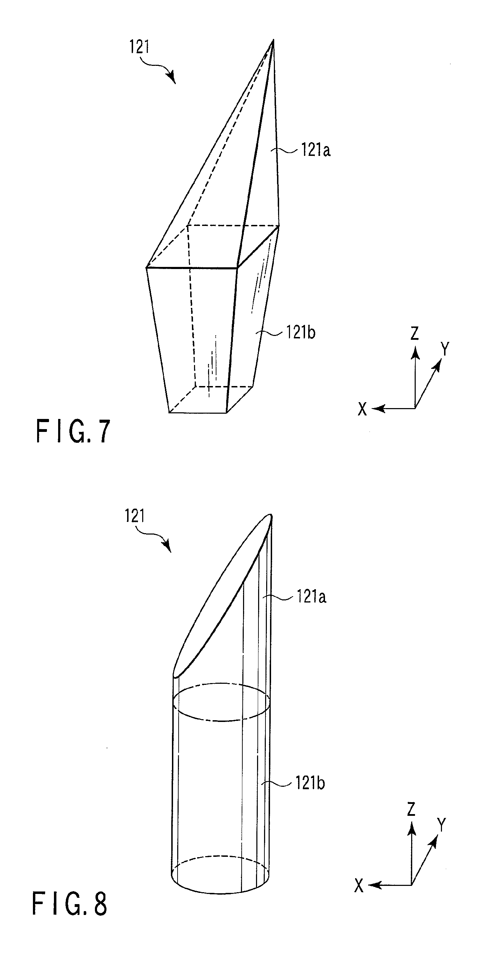

In the micro-needle 121 shown in FIG. 7, the first end section 121a has the structure in which it is tapered down from an end on the side of second end section 121b to another end. The second end section 121b has the structure in which it is tapered down from an end on the side of the first end section 121a to another end. To be more specific, the first end section 121a has an oblique quadrangular pyramid shape. The second end section 121b has a truncated quadrangular pyramid shape.

In the case where such a structure is employed, it is possible to make the inserted micro-needle 121 difficult to be withdrawn from the living body as compared with the case where the structure shown in FIG. 4 is employed. Further, in the case where such a structure is employed, it is possible to easily break the micro-needle 121 in the state that it is inserted into the living body as compared with the case where the structure shown in FIG. 4 is employed. Therefore, this structure is suitable for leaving the micro-needle 121 in the living body. When the micro-needle 121 contains a drug, a longer duration of the pharmacologic effect can be achieved by leaving the micro-needle 121 in the living body.

The micro-needle 121 shown in FIG. 8 has the structure in which it is tapered down from an end on the side of the support layer 11 to another end. The first end section 121a has a truncated circular cylinder shape. The second end section 121b has a circular cylinder shape. When the first end section 121a is a truncated cylinder as above, it is relatively easy to form a sharp tip.

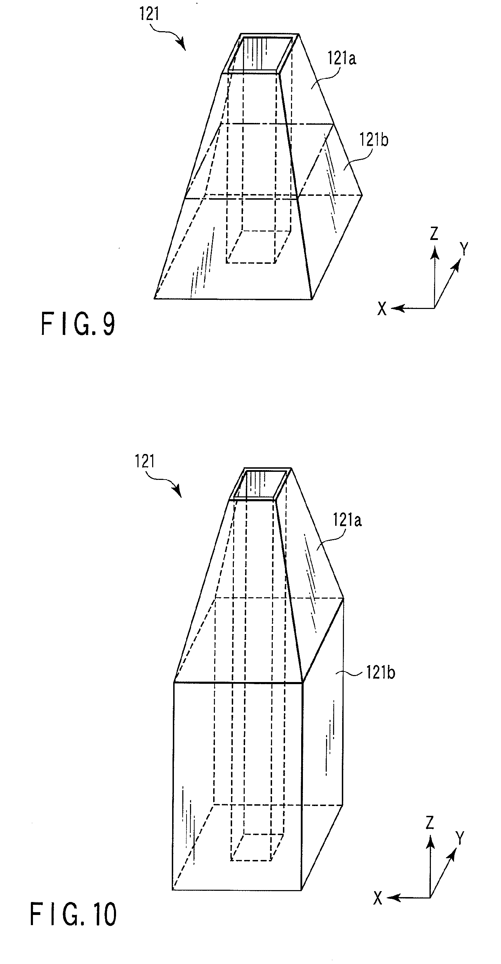

Each of the micro-needles 121 shown in FIGS. 9 and 10 has the structure in which it is tapered down from an end on the side of the support layer 11 to another end and is provided with a through-hole extending in the longitudinal direction. In each micro-needle 121, the first end section 121a has a truncated quadrangular pyramid shape provided with a through-hole extending in the height direction. In the micro-needle 121 shown in FIG. 9, the second end section 121b has a truncated quadrangular pyramid shape provided with a through-hole extending in the height direction. In the micro-needle 121 shown in FIG. 10, the second end section 121b has a quadrangular prism shape provided with a through-hole extending in the height direction.

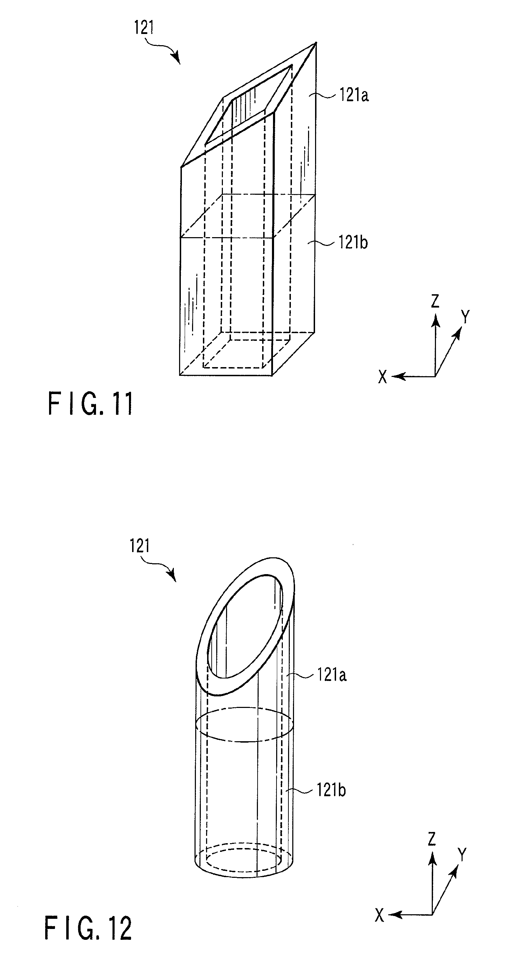

Each of the micro-needles 121 shown in FIGS. 11 and 12 has the structure in which it is tapered down from an end on the side of the support layer 11 to another end and is provided with a through-hole extending in the longitudinal direction. In the micro-needle 121 shown in FIG. 11, the first end section 121a has a truncated quadrangular prism shape provided with a through-hole extending in the height direction, while the second end section 121b has a right quadrangular prism shape provided with a through-hole extending in the height direction. In the micro-needle 121 shown in FIG. 12, the first end section 121a has a truncated circular cylinder shape provided with a through-hole extending in the height direction, while the second end section 121b has a right circular cylinder shape provided with a through-hole extending in the height direction.

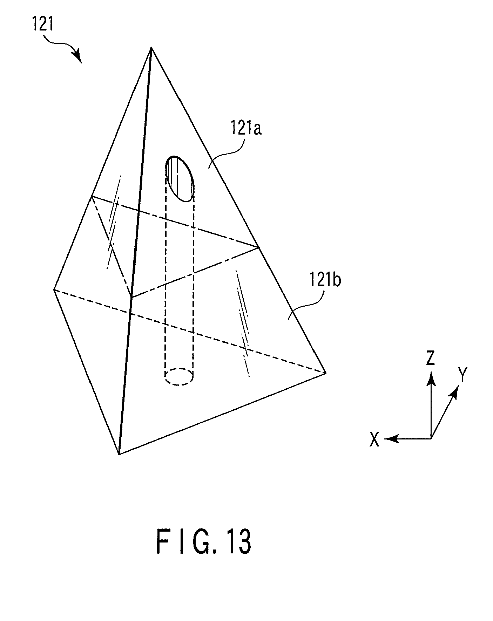

The micro-needle 121 shown in FIG. 13 has the structure in which it is tapered down from an end on the side of the support layer 11 to another end and is provided with a through-hole extending in the height direction. The first end section 121a has a triangular pyramid shape provided with a through-hole extending in the height direction. The second end section 121b has a truncated triangular pyramid shape provided with a through-hole extending in the height direction. In the micro-needle 121 shown in FIG. 13, one of the openings of the through-hole is located at the base of the triangular pyramid, while the other opening is located not at the vertex of the triangular pyramid but at the lateral face of the triangular pyramid.

When the micro-needle 121 is provided with a through-hole as shown in FIGS. 9 to 13, the through-hole can be filled with the feed substance such as the bioactive substance, for example. Thus, in this case, much more amount of the feed substance can be delivered into the living body as compared with the case where the through-hole is omitted.

Note that the micro-needle 121 may be provided with a recess instead of the through-hole. The recess can be filed with the feed substance such as the bioactive substance, for example. Thus, also in this case, much more amount of the feed substance can be delivered into the living body as compared with the case where the through-hole is omitted.

The through-hole formed in the micro-needle 121 can be used as a channel for transferring a substance out of the living body or into the living body. For example, in the case where blood collection or bloodletting is performed, the through-hoe can be used as a channel for transferring the blood out of or into the living body. Alternatively, a liquid substance can be delivered into the living body via the through-hole. When the through-hole is used for such a purpose, the support layer 11 may be provided with a channel that connects the through-hole with the exterior of the micro-needle patch 1.

The micro-needle patch 1 can be manufactured, for example, by the following method.

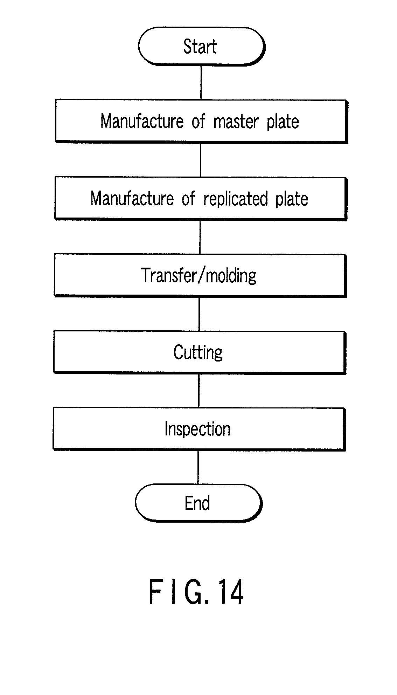

FIG. 14 is a flow-chart showing an example of a method for manufacturing a micro-needle patch.

According to this method, a master plate provide with protrusions is manufactured first. The protrusions are formed such that they have almost the same shapes and are arranged correspondingly with the micro-needles 121.

Next, using the master plate, a plate having recessed pattern corresponding to the protruding pattern is formed. Subsequently, using this plate, a replicated plate having a protruding pattern corresponding to the recessed pattern is formed.

Then, the replicated plate is pressed against a back surface of a film or sheet made of a raw material of the micro-needles 121, and the film or sheet is heated. To do so, the above-described protruding pattern is produced on a surface of the film or sheet. The film or sheet is removed from the replicated plate after cooled down sufficiently.

Next, the molded film or sheet is cut out into appropriate dimensions. Thus, the micro-needle patch 1 is obtained. Note that in ordinary cases, multiple micro-needle patches 1 are manufactured from a single film or sheet.

Then, the micro-needle patches 1 are subjected to an inspection. As above, the manufacture of the micro-needle patches 1 is completed.

In this method, the plate having the protruding pattern is used as a plate for forming a pattern on the film or sheet. Alternatively, as the plate for forming a pattern on the film or sheet, a plate having a recessed pattern or both of a plate having a protruding pattern and a plate having a recessed pattern may be used.

In the case where the feed substance is supported by the surface of the micro-needles 121, the above-described manufacturing process may further includes a step for spraying a fluid including the feed substance toward the micro-needle array 12, for example. In the case where a multilayered structure is employed in the support layer 11, the above-described process may further includes a step for adhering another layer on the film or sheet and/or a step for forming another layer on the film or sheet after the step for transferring the protruding pattern onto the film or sheet.

The film or sheet used in this method can be manufactured, for example, by the following method. First, chitin is dissolved in a methanol solution of calcium compound. Next, a large amount of water is added to the solution so as to precipitate the chitin. Subsequently, calcium is removed from the precipitate by dialysis. Thus, a white gel having a chitin content of about 4 to 5% is obtained. Then, the gel is mixed with distilled water to prepare a suspension, and papermaking using this suspension is performed. Further, a laminar product is subjected to pressing and drying so as to obtain the film or sheet having a chitin content of 100%.

The micro-needle patch 1 can be manufactured by other methods. For example, the micro-needle array 12 may be formed using photolithography. In this case, a photomask that is provided with light-shielding portions corresponding to the micro-needles 121 can be used.

Next, examples of the present invention will be described.

### (Example 1)

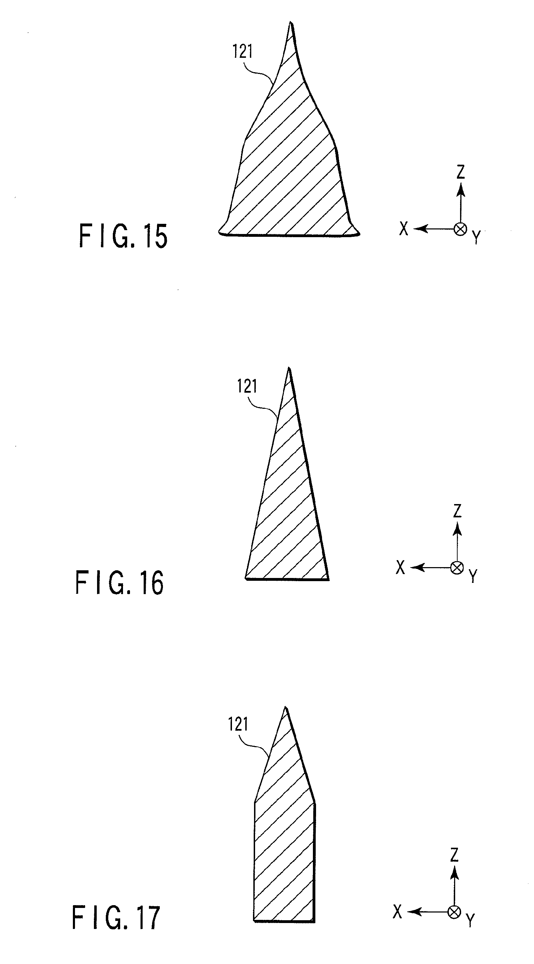

FIGS. 15 to 20 are sectional views schematically showing structures of micro-needles employed in Example 1. Each of the micro-needles 121 shown in FIGS. 15 to 20 has a shape tapering down from one end to another end, and all the cross sections thereof perpendicular to the Z direction are circular. Each of the micro-needles 121 shown in FIGS. 15 to 17 has a symmetry axis parallel with the Z direction. On the other hand, each of the micro-needles 121 shown in FIGS. 1 to 20 does not have a symmetry axis parallel with the Z direction.

In this example, micro-needle patches 1 each having the structure shown in FIG. 1 and differing in the structures of the micro-needles 121 from one another are manufactured by the same method as described with reference to FIG. 14. To be more specific, as a material of the micro-needle patches 1, a mixture of chitin/chitosan and insulin was used. In the mixture, the sum of the chitin content and the chitosan content was set at 70% by mass, while the insulin content was set at 30 by mass. In these micro-needle patches 1, the structures shown in FIGS. 15 to 20 were employed in the micro-needles 121. In each of the micro-needle patches 1, the minimum angle of the tip of the first end section 121a was set at 20°.

The same micro-needle patches were also manufactured using a mixture of maltose and insulin instead of the mixture of chitin/chitosan and insulin.

Then, for each of the micro-needle patches, the performances of the micro-needles were tested using a tension and compression-testing machine "TENSILON (trade mark)". To be more specific, a silicone rubber layer and a micro-needle patch were stacked with a skin of a rat interposed therebetween, and the layered product was mounted on the tension and compression-testing machine. The skin of rat was bought from CHARLES RIVEW JAPAN, INC.

The results of the tests are summarized in TABLE 2 below. Note that in TABLE 2, "Punctured" denotes a proportion of the micro-needles that could be inserted into the skin of a rat. "Broken" denotes a proportion of the broken micro-needles. "Strength" denotes a relative value of the strength supposing the strength to be 100 when chitin/chitosan is used and the structure shown in FIG. 15 is employed.

**TABLE 2**

| Structure | | FIG.15 | FIG.16 | FIG.17 | FIG.18 | FIG.19 | FIG.20 |
|---|---|---|---|---|---|---|---|
| Chitin/ chitosan | Punctured (%) | 60 | 100 | 70 | 50 | 80 | 50 |
| | Broken (%) | 20 | 0 | 15 | 30 | 10 | 30 |
| | Strength (%) | 100 | 150 | 110 | 80 | 120 | 85 |
| Maltose | Punctured (%) | 20 | 30 | 20 | 10 | 20 | 10 |
| | Broken (%) | 70 | 60 | 70 | 90 | 70 | 90 |
| | Strength (%) | 10 | 15 | 10 | 5 | 10 | 5 |

As shown in TABLE 2, chitin/chitosan achieved excellent performances as compared with the maltose.

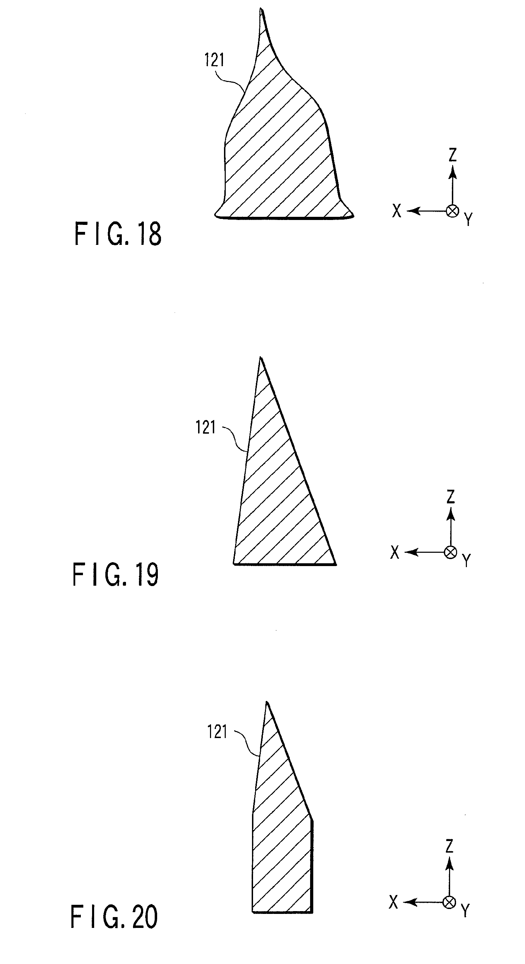

The patch employing the structure shown in FIG. 15 was superior in performances regarding puncture, breaking and strength than the patch employing the structure shown in FIG. 18. This result reveals that micro-needles each having a symmetry axis parallel with the longitudinal direction can achieve superior performances regarding puncture, breaking and strength than micro-needles without such a symmetry axis.

In the case where maltose was used, changing the structure of the micro-needles from the structure shown in FIG. 15 to the structure shown in FIG. 16 achieved 10%, 10% ad 5% increases in the performances regarding puncture, breaking and strength, respectively. On the other hand, in the case where chitin/chitosan was used, changing the structure of the micro-needles from the structure shown in FIG. 15 to the structure shown in FIG. 16 achieved 40%, 20% ad 50% increases in the performances regarding puncture, breaking and strength, respectively. This result reveals that the combination of chitin/chitosan and the structure shown in FIG. 16 produces a synergistic effect. This synergistic effect produced by the chitin/chitosan and the structure of the micro-needles can also be seen when the structure of the micro-needles are changed from the structure shown in FIG. 15 to the structure shown in FIG. 19. This reveals that in the case where chitin/chitosan is used, a cone shape is advantageous to the micro-needles.

### (Example 2)

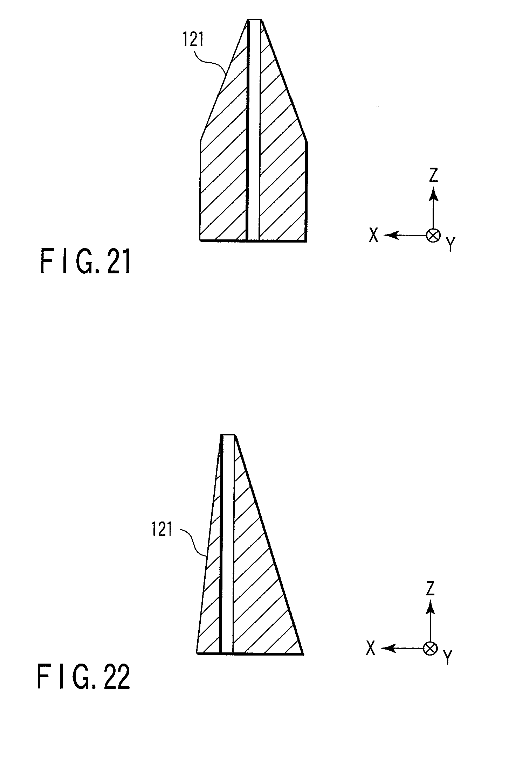

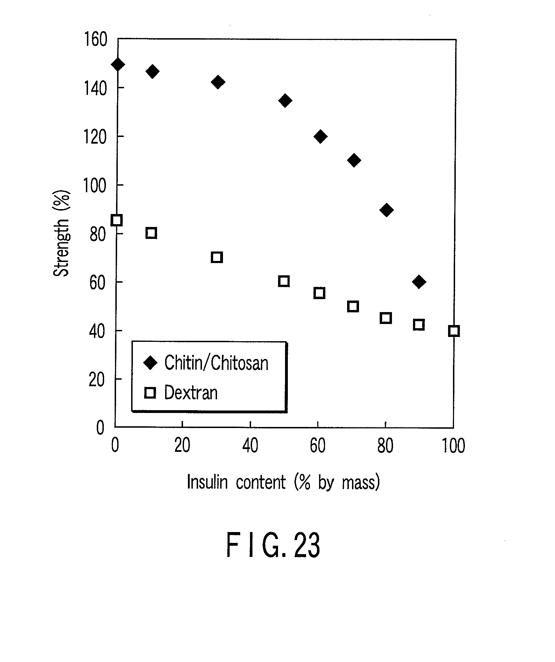

FIGS. 22 and 23 are sectional views schematically showing structures of micro-needles employed in Example 2. Each of the micro-needles 121 shown in FIGS. 22 and 23 has a shape tapering down from one end to another end, and all the cross sections thereof perpendicular to the Z direction are circular. Each of the micro-needles 121 is provided with a through-hole extending in the Z direction. The micro-needle 121 shown in FIG. 21 has a symmetry axis parallel with the Z direction. On the other hand, the micro-needle 121 shown in FIG. 22 does not have a symmetry axis parallel with the Z direction.

In this example, micro-needle patches made of a mixture of chitin/chitosan and insulin were manufactured by the same method as in Example 1 except that the structures shown in FIGS. 21 and 22 were employed in micro-needles. Then, the same test as described in Example 1 were performed on the micro-needle patches. As a result, in the case where the structure shown in FIG. 21 was employed in the micro-needles, achieved were performances similar to those achieved in Example 1 when chitin/chitosan was used and the structure shown in FIG. 17 was employed. On the other hand, in the case where the structure shown in FIG. 22 was employed in the micro-needles, achieved were performances similar to those achieved in Example 1 when chitin/chitosan was used and the structure shown in FIG. 19 was employed.

### (Example 3)

In this example, micro-needle patches differing in insulin contents from one another were manufactured by the same method as in Example 1. Then, the strengths of the micro-needles were determined on each of the micro-needle patches by the same method as described in Example 1. The results are shown in FIG. 23.

FIG. 23 is a graph showing the relationship between the insulin content and the strength of a micro-needle. In the figure, the abscissa denotes the insulin content, while the ordinate denotes the strength of the micro-needles.

As shown in FIG. 23, in the case where chitin/chitosan was used and the insulin content was about 50% or less, the strength of 135% or more was achieved. Also, it was seen that in this case, significantly high performances were achieved as compared with the case where maltose was used and the synergistic effect was produced.

Then, the same tests were performed using ketamine as an anesthetic instead of insulin. As a result, in the case where chitin/chitosan was used and the ketamine content was 30% or less, the strength of 140% or more was achieved. The same result was also obtained in the case where chitin/chitosan was used and vaccine was used instead of insulin.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A micro-needle comprising first and second end sections arranged in a longitudinal direction, and made of a biocompatible and biodegradable material including chitin and/or chitosan.

2. The micro-needle according to claim 1, wherein the micro-needle is tapered down from an end to another end.

3. The micro-needle according to claim 2, wherein the micro-needle has a symmetry axis parallel with the longitudinal direction.

4. The micro-needle according to claim 2, wherein the first end section has a cone shape and the second end section has a cylindrical shape.

5. The micro-needle according to claim 3, wherein the first end section has a right cone shape and the second end section has a right cylindrical shape.

6. The micro-needle according to claim 1, wherein the micro-needle has a cone shape.

7. The micro-needle according to claim 1, wherein the micro-needle has a right cone shape.

8. The micro-needle according to claim 1, a sum of a chitin content and a chitosan content in the biocompatible and biodegradable material is equal to or greater than 50% by mass.

9. The micro-needle according to claim 1, wherein the biocompatible and biodegradable material further includes a biologically active substance.

10. The micro-needle according to claim 1, wherein the micro-needle is provided with a through-hole or recess extending in the longitudinal direction.

11. A micro-needle patch, comprising:
a support layer with first and second main surfaces; and
micro-needles each extending from the first main surface, each of the micro-needles being the micro-needle according to one of claims 1 to 10 supported by the first main surface at an end of the second end section.

12. The micro-needle patch according to claim 11, further comprising a biologically active substance supported by a surface of at least one of the first and second end sections.

13. The micro-needle patch according to claim 12, wherein the first main surface includes chitin and/or chitosan.
